(19) Europäisches Patentamt
European Patent Office
Office européen des brevets

(11) EP 1 749 513 B1

(12) # EUROPÄISCHE PATENTSCHRIFT

(45) Veröffentlichungstag und Bekanntmachung des Hinweises auf die Patenterteilung:
**04.03.2009 Patentblatt 2009/10**

(51) Int Cl.:
***A61K 6/083*** *(2006.01)* ***C07F 9/53*** *(2006.01)*

(21) Anmeldenummer: **05107106.6**

(22) Anmeldetag: **01.08.2005**

(54) **Photopolymerisierbare Dentalmaterialien mit Bisacylphosphinoxiden als Initiator**

Photopolymerisable dental material with bisacylphosphine oxides as initiator

Matériau dentaire photopolymérisable avec les oxides de bisacylphosphine comme initiateur

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**07.02.2007 Patentblatt 2007/06**

(73) Patentinhaber: **Ivoclar Vivadent AG**
**9494 Schaan (LI)**

(72) Erfinder:
- **Moszner, Prof. Dr. Norbert**
  **9495, Triesen (LI)**
- **Salz, Dr. Ulrich**
  **88131, Lindau (DE)**
- **Rheinberger, Dr. Volker**
  **9490, Vaduz (LI)**
- **Gruber, Prof. Dr. Heinrich**
  **1190, Wien (AT)**
- **Liska, Dr. Robert**
  **1200, Wien (AT)**
- **Ganster, Beate**
  **1020 Wien (AT)**
- **Ullrich, Gerald**
  **7035, Stenbrunn (AT)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 1 236 459**
**WO-A-03/019295**
**WO-A-03/068785**
**DE-A1- 3 443 221**
**DE-A1- 3 801 511**
**DE-A1- 19 532 358**
**DE-A1- 19 708 294**

- **DATABASE WPI Section Ch, Week 200020 Derwent Publications Ltd., London, GB; Class A96, AN 2000-232897 XP002361029 & JP 2000 053519 A (TOKUYAMA SODA KK) 22. Februar 2000 (2000-02-22)**
- **DATABASE WPI Section Ch, Week 200016 Derwent Publications Ltd., London, GB; Class A12, AN 2000-176842 XP002361030 & JP 2000 026226 A (TOKUYAMA SODA KK) 25. Januar 2000 (2000-01-25)**
- **RICHARDS, N.D.; DICKENS, S.H.; ANTONUCCI, J.M.: "Dental polymeric composites activated with camphorquinone or diacyl phosphine oxide photoinitiators" POLYMER PREPRINTS, Bd. 45, Nr. 2, 2004, Seiten 362-363, XP008057791**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft polymerisierbare Dentalmaterialien wie Adhäsive, Beschichtungen, Zemente und Komposite, die Bisacylphosphinoxide als Photoinitiatoren enthalten.

Eine Vielzahl radikalisch polymerisierbarer Dentalmaterialien, wie z.B. Versiegler, Dentin- und Schmelz-Adhäsive, Befestigungsmaterialien und Füllungskomposite werden vorwiegend durch Bestrahlung mit Licht zur Aushärtung gebracht. Der Grund dafür ist die einfache Handhabbarkeit lichthärtender Materialien. Sie sind meist einkomponentig, d.h. sie müssen vor der Anwendung nicht angemischt werden. Weiter zeigen sie eine lange Verarbeitungszeit und härten dann, wenn gewünscht bei Bestrahlung schnell aus. Auch zeichnen sie sich durch eine gute Lagerstabilität bei Raumtemperatur aus.

Aus Gründen der Gewebeverträglichkeit und der ausreichenden Durchhärtung bei pigmentierten Systemen, wird hauptsächlich mit Licht im Wellenlängenbereich von 400 bis 500 nm bestrahlt. Eines der ersten in radikalisch polymerisierbaren Dentalmaterialien eingesetzten Photoinitiatorsysteme war die Kombination aus einem $\alpha$-Diketon und einem Amin wie in GB 1 408 265 beschrieben. Entsprechende Dentalzusammensetzungen, die dieses Photoinitiatorsystem enthalten, werden z.B. in der US 4,457,818 und der US 4,5252,56 offenbart, wobei vorzugsweise Campherchinon als $\alpha$-Diketon eingesetzt wird. Es sind aber auch andere Diketone eingesetzt worden, wie z.B. Kombinationen von 1-Aryl-2-alky-1,2-ethandionen mit cyclischen Diketonen (US 6, 204, 302).

Einkomponentige Photoinitiatoren, sogenannte $\alpha$-Spalter wie Titanocene, Acylphosphonate, Acylphosphinoxide oder Bisacrylphosphinoxide, finden in lichthärtenden Dentalmaterialen ebenfalls Einsatz. Titanocene sind allein jedoch nicht besonders reaktiv und werden vorzugsweise in Kombination mit Aminen und/oder Peroxiden angewendet (EP 0 334 338). Auch Acylphosphonate wie z.B. das Benzoyl-di(2,6-dimethylphenyl)phosphonat werden wegen ihrer geringen Durchhärtungstiefe bevorzugt in Kombination mit einem zweiten Initiatorsystem, wie z.B. dem Campherchinon/Aminsystem, angewendet (EP 0 336 417 A2).

Die EP 0173 567 A2 offenbart Dentalkomposite, die als Initiatoren für die radikalische Polymerisation Acylphosphinoxide enthalten, wie z.B. das in DE 29 09 992 A1 beschriebene 2,4,6-Trimethylbenzoyl-diphenylphosphinoxid.

Die EP 1 236 459 A1 beschreibt ein dentales Komposit, das Acyl- oder Bisacylphosphinoxide als Photoinitiatoren enthält. Die Materialien enthalten eine spezielle Mischung aus Füllstoffpartikeln, die den Werkstoffen nach der Härtung einen verbesserten Oberflächenglanz verleihen sollen.

In der EP 0 948 955 A1 werden dentale Zusammensetzungen offenbart, die als Initiator eine Kombination aus Acylphosphinoxid, organischem Peroxid, tertiärem Amin und aromatischer Sulfinsäure enthalten. Als Monomere können unter anderem acide Monomere verwendet werden.

Die US 2002/0035169 A1 beschreibt eine antibakterielle Adhäsivzusammensetzung, bei der als Initiatorsystem bevorzugt eine Mischung aus Acylphosphinoxid und einem $\alpha$-Diketon eingesetzt wird.

Die DE 34 43 221 A1 offenbart Bisacylphosphinoxide, die sich als Initiatoren für die Photopolymerisation von Verbindungen mit C=C-Bindungen eignen sollen.

Aus der DE 38 01 511 A1 sind photopolymerisierbare Dentalmassen bekannt, die in zwei Schritten ausgehärtet werden können. Diese Massen enthalten eine erste Photoinitiatorkomponente mit einem Absorptionsmaximum von < 450 nm und eine zweite Photoinitiatorkomponente mit einem Absorptionsmaximum von > 450 nm. Als erste Photoinitiatorkomponente werden Bisacylphosphinoxide und als zweite Photoinitiatorkomponente $\alpha$-Diketone eingesetzt.

Die DE 38 37 569 A1 betrifft photopolymerisierbare Dentalmassen, die Bisacylphosphinoxide als Photoinitiator für die Thiol-En-Polymerisation enthalten.

Die US 5,399,770 offenbart die Verwendung von Alkylbisacylphosphineoxiden als Photoinitiatoren für Dentalmaterialien auf der Basis ethylenisch ungesättigter Verbindungen. Die Photoinitiatoren sollen leicht herzustellen sein.

Die DE 195 32 358 A1 offenbart die Verwendung von alkoxyphenyl-substituierten Bisacylphosphinoxiden als Photoinitiatoren zur Photopolymerisation, die im Bereich von ca. 200 bis ca. 600 nm aktiv sind.

Die WO 03/019295 A1 offenbart bathochrome Mono- und Bisacylphosphinoxide, die sich als Photoinitiatoren für olefinisch ungesättigte Monomere eignen.

Photoinitiatorsysteme, die aus einem $\alpha$-Diketon und einem Amin aufgebaut sind, sind für den Einsatz in selbstätzenden, selbstkonditionierenden Dentalmaterialien nur bedingt tauglich. Derartige Dentalmaterialien enthalten in der Regel acide Monomere, die in der Lage sind, die Zahnhartsubstanz anzuätzen, so daß eine Präkonditionierung der Zahnhartsubstanz mit Säure nicht notwendig ist. $\alpha$-Diketon/Amin-Photoinitiatorsysteme werden jedoch durch die aciden Monomere protoniert und verlieren dadurch an Effizienz.

Photoinitiatoren, wie die Acyl- und Bisacylphosphinoxide, die durch monomolekulare Bindungsspaltung, sogenannte Norrish-Typ I-Spaltung, polymerisationsauslösende Radikale bilden, sind unter sauren Bedingungen zwar besser geeignet, haben aber den Nachteil, daß sie in wäßrigen Systemen nur wenig löslich sind. Außerdem sind im Vergleich zu $\alpha$-Diketon/Amin-Photoinitiatorsystemen meist höhere Initiatorkonzentrationen erforderlich, so daß die Gefahr besteht, daß nach Aushärtung der Materialien nicht umgesetzte und damit auswaschbare Photoinitiatoranteile vorliegen, was unter toxikologischen Gesichtspunkten nachteilig ist. Weiterhin ist von den Acylphosphinoxiden bekannt,

daß die Kohlenstoff-Phosphor-Bindung leicht durch nucleophile Verbindungen, wie z.B. Wasser oder Alkohole gespalten wird. Dadurch wird der Photoinitiator sukzessive abgebaut, was einen Aktivitätsverlust der Initiatoren und damit eine unvollständige Härtung des Restaurationsmaterials zur Folge hat. Dies bedeutet, daß die Dentalmaterialien bei Lagerung mit der Zeit ihre klinische Tauglichkeit verlieren.

Der Erfindung liegt demgemäß die Aufgabe zugrunde, Photoinitiatoren für Dentalmaterialien bereitzustellen, die im sichtbarem Bereich polymerisationsauslösend sind, eine gute Löslichkeit in wäßrigen Systemen aufweisen und in Gegenwart von Wasser und Säuren stabil sind.

Diese Aufgabe wird erfindungsgemäß durch polymerisierbare Dentalwerkstoff gelöst, die mindestens ein radikalisch polymerisierbares Monomer und mindestens ein Bisacylphosphinoxid der Formel I enthalten,

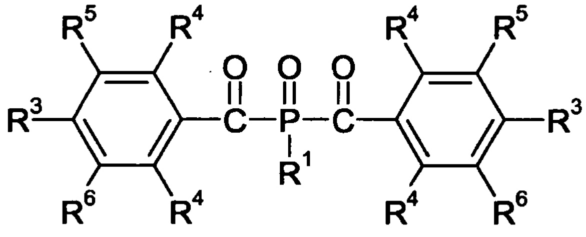

Formel (I)

in der die Variablen unabhängig voneinander die folgenden Bedeutungen haben:

$R^1$ = ein linearer oder verzweigter $C_2$ bis $C_{14}$-Alkylrest, der durch ein oder mehrere O-Atome unterbrochen sein kann, PG-Y-$R^2$-X-, ein substituierter oder unsubstituierter, aromatischer $C_6$ bis $C_{14}$-Rest;

$R^2$ = entfällt, ein linearer oder verzweigter $C_1$- bis $C_{20}$-Alkylenrest, der durch ein oder mehrere O-Atome unterbrochen sein kann,

$R^3$ = H, ein linearer oder verzweigter $C_1$- bis $C_6$-Alkylrest oder PG-Y-$R^2$-X-,

$R^4$ = ein linearer oder verzweigter $C_1$-$C_6$-Alkyl- oder -O-$C_1$-$C_6$-Alkylrest,

$R^5$ = H oder PG-Y-$R^2$-X-,

$R^6$ = H oder PG-Y-$R^2$-X-,

PG = eine polymerisationsfähige Gruppe,

X = entfällt, O oder S,

Y = entfällt, O, S, eine Ester-, Amid- oder Urethangruppe,
wobei das Bisacylphosphinoxid mindestens eine PG-Y-$R^2$-X-Gruppe aufweist und wobei X und/oder Y entfällt wenn $R^2$ entfällt.

Der Hinweis, daß Gruppen durch Sauerstoffatome unterbrochen sein können, ist so zu verstehen, daß Sauerstoffatome in die Kohlenstoffkette der Gruppen eingeschoben werden, d.h. beidseitig von Kohlenstoffatomen gebunden werden. Die Sauerstoffatome können somit keine endständige Position einnehmen. Werden mehrere Atome in eine Kohlenstoffkette integriert, müssen sie jeweils durch mindestens ein Kohlenstoffatom voneinander getrennt sein. Unter "Kohlenstoffkette" werden keine ringförmigen Molekülgruppen verstanden. Die Gesamtzahl der Atome, die in die Kohlenstoffkette integriert wird, ist mindestens um den Wert 1 kleiner als die Zahl der Kohlenstoffatome in der Kette.

Die bei $R^1$ gegebenenfalls vorhandenen Substituenten sind vorzugsweise aus $C_1$- bis $C_{15}$-Alkoxyresten, die durch ein oder mehrere O-Atome unterbrochen sein können, PG-Y-$R^2$-X-, Thiomethyl-, Dimethylamino- und/oder Diethylaminogruppen, besonders bevorzugt aus $C_1$- bis $C_6$-Alkoxyresten, die durch ein oder mehrere O-Atome unterbrochen sein können, und PG-Y-$R^2$-X- ausgewählt. Die aromatischen Reste könne ein oder mehrfach, vorzugsweise 1 bis 2-fach substituiert sein.

Bevorzugte polymerisationsfähige Gruppen sind die Vinyl-, Allyl-, (Meth)acryloyl- und/oder Vinylcyclopropylgruppe.

Bevorzugt sind Verbindungen der Formel (I) bei denen X entfällt und $R^2$ ein linearer oder verzweigter $C_1$- bis $C_{20}$-Alkylenrest ist, der durch ein oder mehrere O-Atome unterbrochen sein kann.

Bevorzugte Bedeutungen der Variablen, die unabhängig voneinander ausgewählt werden können, sind:

- $R^1$ Phenyl, das unsubstituiert oder durch PG-Y-$R^2$-X- substituiert ist;
- $R^2$ $-[CH_2]_n-[-O-CH_2-CH_2]_m-$ mit n = 1, 2, 3 oder 4 und m = 0, 1,2 oder 3;
- $R^3$ H, $C_1$-$C_3$-Alkyl oder PG-Y-$R^2$-X-;
- $R^4$ $C_1$-$C_3$-Alkyl oder -O-$C_1$-$C_3$-Alkyl;
- $R^5$ H oder PG-Y-$R^2$-X-;
- $R^6$ H oder PG-Y-$R^2$-X-;
- PG $-CH_2-CH=CH_2$ oder $-CH=CH_2$;
- X entfällt;
- Y entfällt oder O.

Im Fall m = 0 ist Y vorzugsweise O.

Das Bisacylphosphinoxid der Formel (I) weist vorzugsweise 1 bis 5, besonders bevorzugt 1 bis 3 PG-Y-$R^2$-X-Gruppen auf.

Die funktionalisierten Bisacylphosphinoxide der allgemeinen Formel (I) lassen sich durch für Bisacvlphosphinoxide bekannte mehrstufige Syntheseverfahren herstellen. Beispielsweise werden in den ersten Synthesestufen geeignet substituierte Benzoylchloride, z.B. durch Veretherung entsprechend substituierter Brombenzoesäure und anschließende Umwandlung in das Säurechlorid mit Thionylchlorid, hergestellt, die dann im zweiten Schritt mit einem geeignet substituierten Dilithiumalkyl- oder -arylphosphin umgesetzt und die gebildeten Produkte dann weiter mit Wasserstoffperoxid zum Bisacylphosphinoxid der Formel (I) oxidiert werden:

1. Stufe:

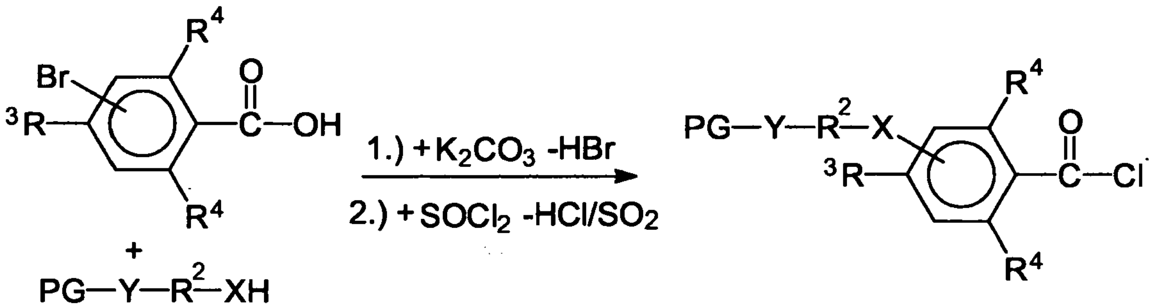


2. und 3. Stufe:

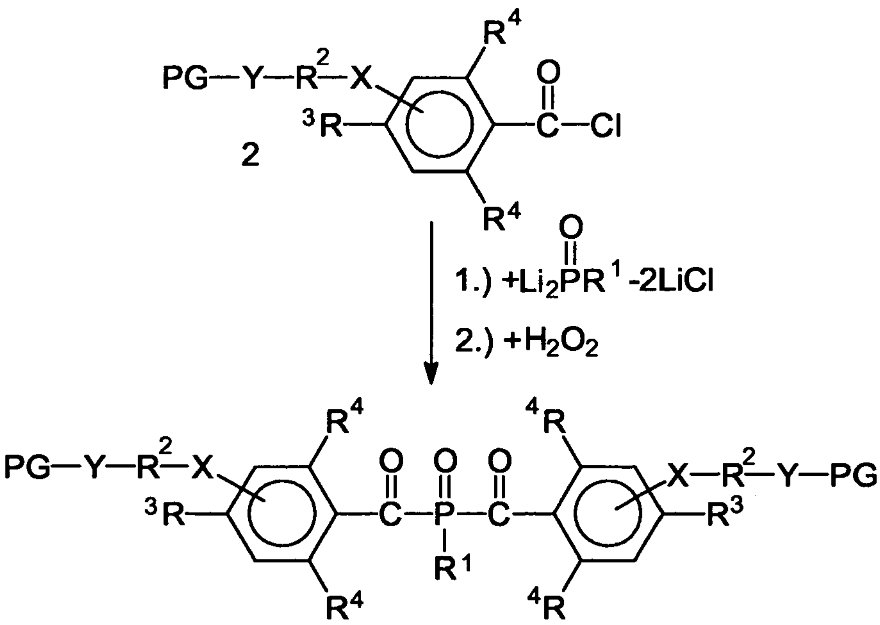


Konkretes Beispiel:

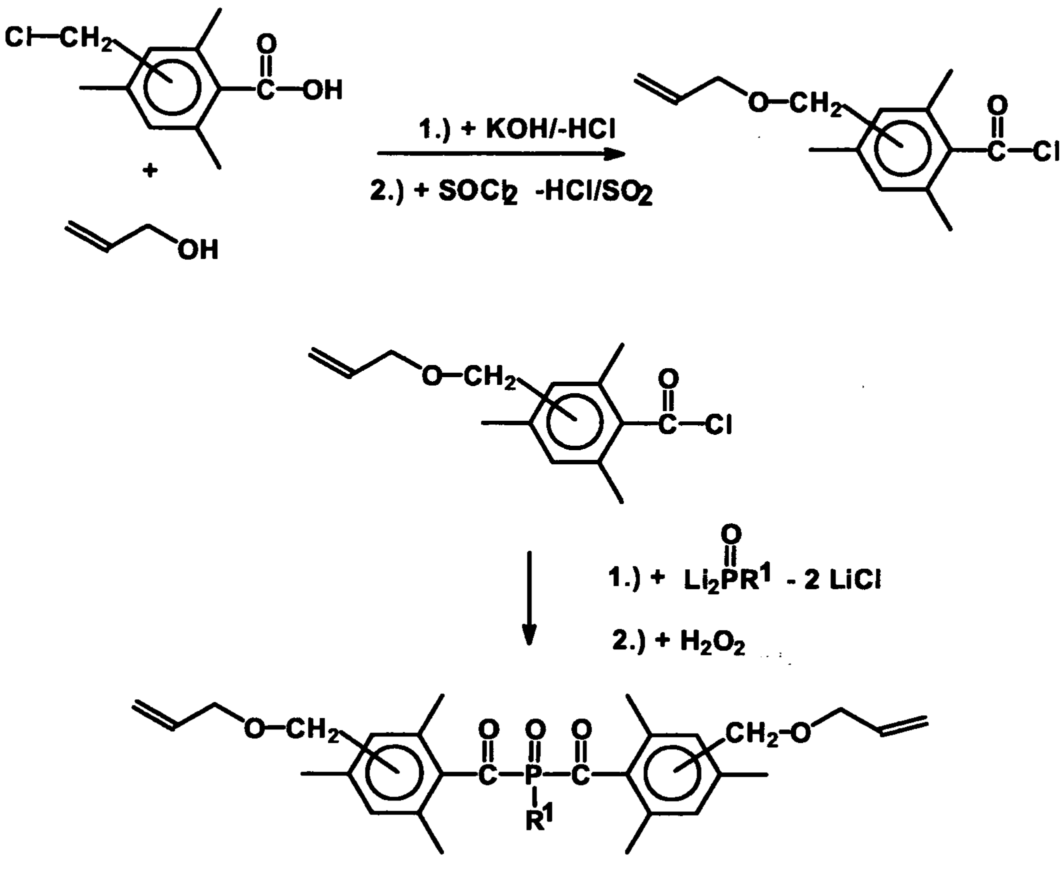


Nachfolgend sind bevorzugte Beispiele der erfindungsgemäßen Bisacylphosphinoxide aufgeführt: Bis-(3-allyloxymethyl-2,4,6-trimethyl-benzoyl)-phenylphosphin-oxid:

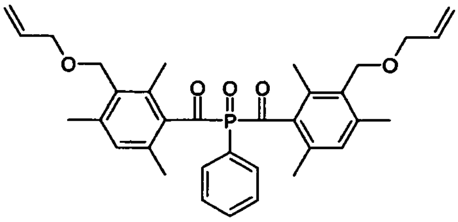

Bis-[3-(2-allyloxy-ethoxymethyl)-2,4,6-trimethylbenzoyl]-phenylphosphinoxid:

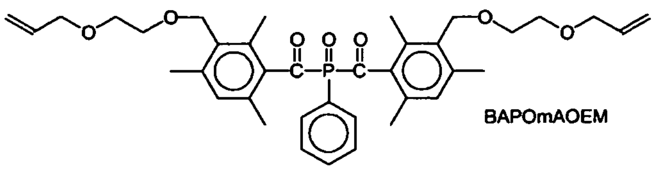

BAPOmAOEM

Bis-{3-[2-(2-allyloxy-ethoxy)-ethoxymethyl]-2,4,6-trimethylbenzoyl}-phenylphosphinoxid:

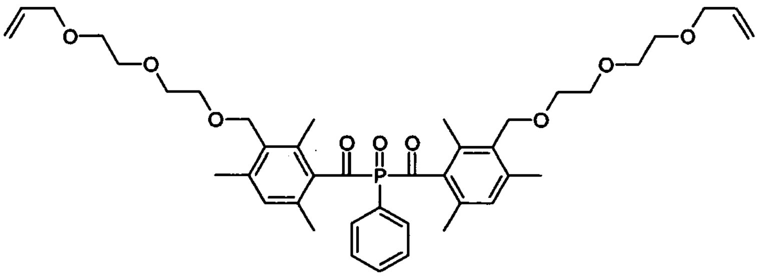

Bis-(3-allyloxymethyl-2,6-dimethoxy-4-methyl-benzoyl)-phenylphosphinoxid:

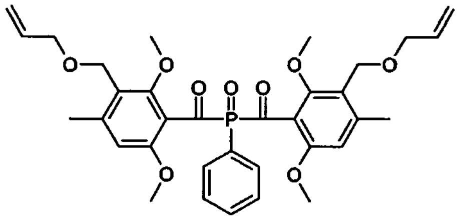

Bis-{[3-(2-allyloxy-ethoxymethyl)-2,6-dimethoxy-4-methyl-benzoyl}-phenylphosphinoxid:

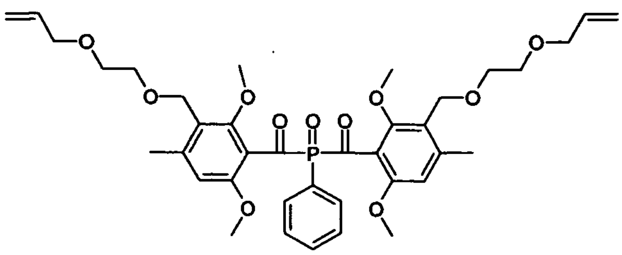

Bis-{3-[2-(2-allyloxy-ethoxy)-ethoxymethyl]-2,6-dimethoxy-4-methyl-benzoyl}-phenylphosphinoxid:

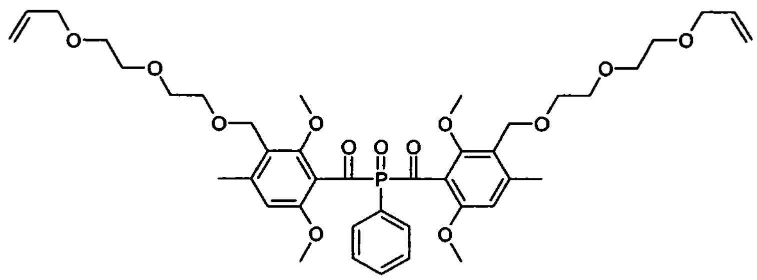

Bis-{3,5-[Bis-(allyloxymethyl)-2,4,6-trimethyl-benzoyl]}-phenylphosphin:

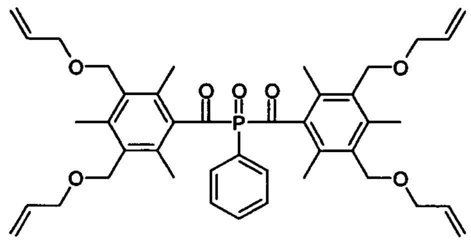

Bis-{3,5-[bis-(2-allyloxy-ethoxymethyl)-2,4,6-trimethylbenzoyl]}-phenylphosphinoxid:

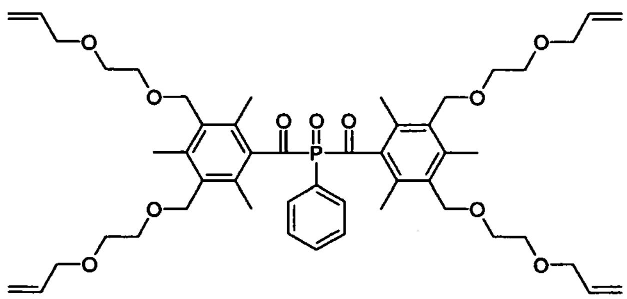

Bis-{3,5-bis-[2-(2-allyloxy-ethoxy)-ethoxymethyl]-2,4,6-trimethyl-benzoyl}-phenylphosphinoxid:

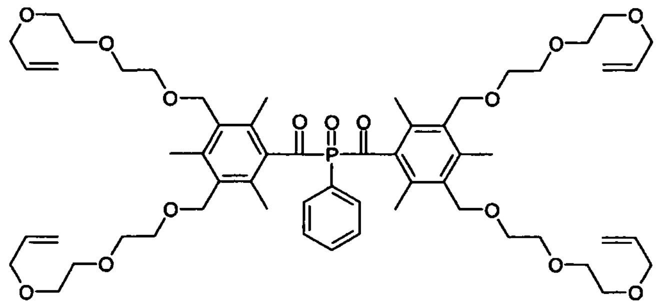

Bis-{3,5-[bis-(allyloxymethyl)-2,6-dimethoxy-4-methyl-benzoyl]}-phenylphosphin:

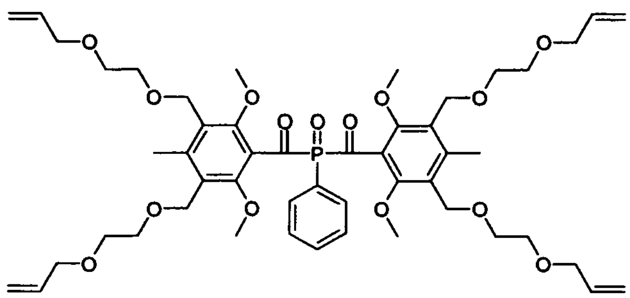

Bis-{3,5-[bis-(2-allyloxy-ethoxymethyl)-2,6-dimethoxy-4-methyl-benzoyl]}-phenylphosphinoxid:

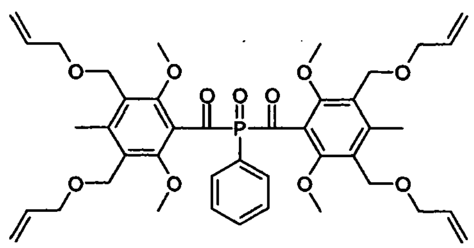

Bis-{3,5-bis-[2-(2-allyloxy-ethoxy)-ethoxymethyl]-2,6-dimethoxy-4-methyl-benzoyl}-phenylphosphinoxid:

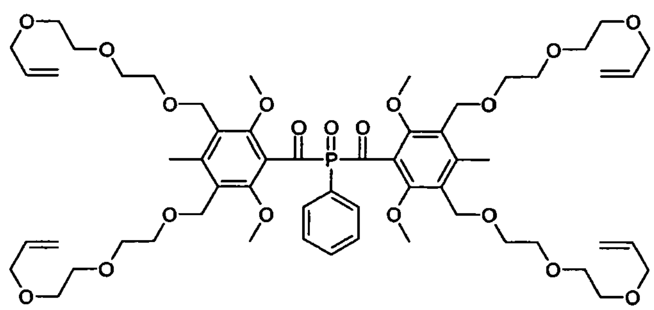

Bis-[2,4,6-trimethyl-benzoyl]-{4-[2-(2-allyloxy-ethoxy)-ethoxymethyl]-phenyl}-phosphinoxid:

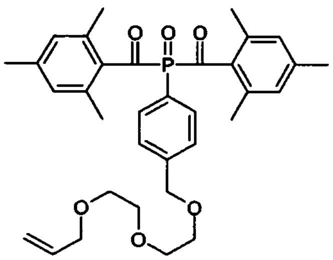

Bis-[2,4,6-trimethyl-benzoyl]-[4-(2-allyloxy-ethoxymethyl)-phenyl]-phosphinoxid:

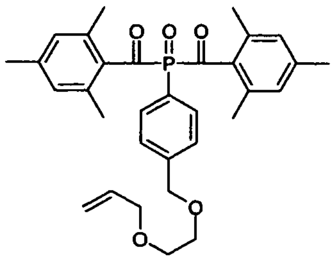

Bis-[2,4,6-trimethyl-benzoyl]-[4-allyloxymethyl-phenyl]-phosphinoxid:

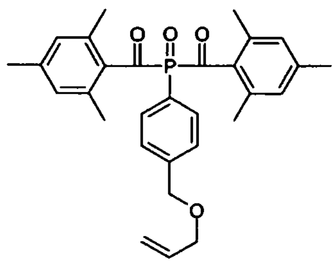

Bis-[2,6-dimethoxy-benzoyl]-[4-allyloxymethyl-phenyl]-phosphinoxid:

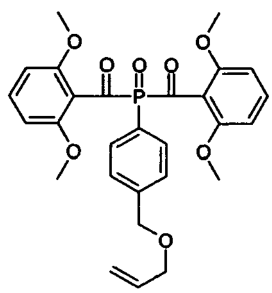

Bis-[2,6-dimethoxy-benzoyl]-[4-(2-allyloxy-ethoxymethyl)-phenyl]-phosphinoxid:

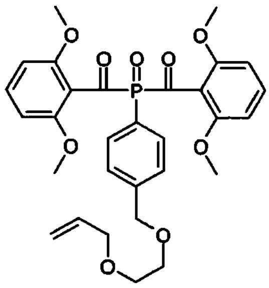

Bis-[2,6-dimethoxy-benzoyl]-{4-[2-(2-allyloxy-ethoxy)-ethoxymethyl]-phenyl}-phosphinoxid:

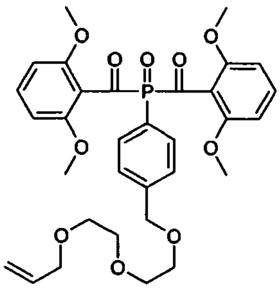

Die Bisacylphosphinoxide der Formel (I) absorbieren im UV-Bereich und zeigen auch eine Absorption größer 400 nm, so daß deren Photolyse und Radikalbildung durch Bestrahlung mit im Dentalbereich üblichen Halogenlampen bzw. auch mit LED-Lampen induziert werden kann. Die Bisacylphosphinoxide der Formel (I) enthalten polymerisationsfähige Gruppen und lassen sich z.B. durch Copolymerisation in Polymere einbauen. Weiterhin sind die erfindungsgemässen Bisacylphosphinoxide sehr gut in polaren organischen Lösungsmitteln, wie Aceton, Ethanol, Acetonitril oder Tetrahydrofuran (THF) oder deren Mischungen mit Wasser löslich, d.h. die Löslichkeit beträgt mindestens 0,01 mmol/g. Dabei ist besonders vorteilhaft, das erfindungsgemässen Bisacylphosphinoxide eine im Vergleich zu bekannten Bisacrylphosphinoxiden verbesserte Lagerstabilität zeigen. Die Verbindungen zeichnen sich durch eine hohe Hydrolysestabilität aus, d.h. sie werden in einer Mischung aus $CH_3CH:H_2O$ (60:40) bei 42 °C in 60 Tagen zu nicht mehr als 15 % hydrolysiert.

Die erfindungsgemässen Dentalwerkstoffe können als radikalisch polymerisierbare Monomere mono- oder mehrfach funktionelle (Meth)acrylate enthalten. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden. Mehrfach funktionelle Monomere haben vernetzende Eigenschaften.

Bevorzugte monofunktionelle (Meth)acrylate sind kommerziell verfügbare monofunktionelle Monomere, wie Methyl-, Ethyl-, Butyl-, Benzyl-, Furfuryl- oder Phenyl(meth)acrylat sowie 2-Hydroxyethyl- oder -propyl(meth)acrylat.

Besonders bevorzugt sind hydrolysestabile Monomere wie hydrolysestabile Mono(meth)acrylate, z.B. Mesitylmethacrylat oder 2-(Alkoyxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, und N-monosubstituierte Methacrylamide, wie z.B. N-Ethylmethacrylamid oder N-(2-Hydroxyethyl)methacrylamid sowie außerdem N-Vinylpyrrolidon und Allylether. Diese Monomere sind bei Raumtemperatur flüssig und eignen sich daher auch als Verdünner.

Bevorzugte mehrfach funktionelle Monomere sind Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethy-lenglycoldi(meth)acrylat, Trimethylolpropantri(meth) acrylat, Pentaerythrittetra(meth)acrylat, sowie Butandioldi(meth)-acrylat, 1,10-Decandioldi(meth)acrylat oder 1,12-Dodecandiol-di(meth)acrylat.

Besonders bevorzugt sind hydrolysestabile Vernetzermonomere, wie z.B. Urethane aus 2-(Hydroxymethyl) acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N'-Diethyl-1,3-bis-(acrylamido)-propan, 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechorid synthetisiert werden können.

Die erfindungsgemäßen Dentalwerkstoffe enthalten vorzugsweise auch mindestens ein radikalisch polymerisierbares, säuregruppenhaltiges Monomer. Bevorzugte Säuregruppen sind Carbonsäuregruppen, Phosphonsäuregruppen, Phosphatgruppen und/oder Sulfonsäuregruppen, wobei Gruppen mit mehr als einem aciden Wasserstoffatom teilweise verestert sein können. Besonders bevorzugt sind Monomere mit Phosphonsäuregruppen oder Phosphatgruppen. Die Monomere können eine oder mehrere acide Gruppen aufweisen, bevorzugte sind Verbindungen mit 1 bis 2 aciden Gruppen.

Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Phosphonsäuremonomere sind Alkenphosphonsäuren, Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methylpentyl-phosphonsäure 2-[4-(Dihydroxyphosphoryl)-2-oxa-butyl]-acrylsäure und 2-[2-Dihydroxyphosphoryl)-ethoxymethyl]-acrylsäure-2,4,6-trimethyl-phenylester.

Bevorzugte acide polymerisationsfähige Phosphorsäureester (Phosphate) sind 2-Methacryloyloxypropylmono- und -dihydrogenphosphat, 2-Methacryloyloxyethylmono- und -dihydrogenphosphat, 2-Methacryloyloxyethyl-phenyl-hydrogenphosphat, Dipentaerythritol-pentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Weiterhin können die Dentalwerkstoffe zur Verbesserung der mechanischen Eigenschaften oder zur Einstellung der Viskosität organische oder anorganische partikuläre Füllstoffe enthalten. Bevorzugte anorganische partikuläre Füllstoffe sind amorphe kugelförmige Materialien auf der Basis von Oxiden, wie $ZrO_2$ und $TiO_2$, nanopartikuläre oder mikrofeine Füllstoffe, wie pyrogene Kieselsäure nanopartikuläres $Al_2O_3$, $Ta_2O_5$, $Yb_2O_3$, $ZrO_2$, Ag oder $TiO_2$ bzw. Mischoxide aus $SiO_2$, $ZrO_2$ und/oder $TiO_2$. oder Fällungskieselsäure sowie Minifüllstoffe, wie Quarz-, Glaskeramik- oder Glaspulver mit einer durchschnittlichen Teilchengrösse von 0,01 bis 1 μm sowie röntgenopake Füllstoffe, wie Ytterbiumtrifluorid oder nanopartikuläres Bariumsulfat. Besonders geeignet sind Füllstoffe, die mit polymerisationsfähigen Gruppen oberflächenmodifiziert sind.

Außerdem können die erfindungsgemässen Dentalwerkstoffe ein oder mehrere weitere Additive enthalten, die aus Stabilisatoren, Aromastoffen, antimikrobiellen Wirkstoffen, fluoridionenabgebenden Additiven, optische Aufhellern, Weichmachern und/oder UV-Absorbern ausgewählt sind.

Die Dentalwerkstoffe eignen sich z.B. als Füllungsmaterialien und besonders als Beschichtungsmaterialien, Adhäsive, selbsthaftende und/oder selbstkonditionierende Befestigungszemente.

Die erfindungsgemäßen Dentalwerkstoffe enthalten vorzugsweise:

a) 0,001 bis 5 Gew.-%, bevorzugt 0,01 bis 3,0 Gew.-% und besonders bevorzugt 0,1 bis 2,0 Gew.-% Bisacylphosphinoxid der Formel (I);

b) 5 bis 80 Gew.-%, bevorzugt 5 bis 60 Gew.-% und besonders bevorzugt 5 bis 50 Gew.-% mono- oder multifunktionelles Monomer;

c) 1 bis 60 Gew.-%, bevorzugt 5 bis 50 Gew.-% und besonders bevorzugt 5 bis 45 Gew.-% acides radikalisch polymerisierbares Monomer;

d) 0 bis 80 Gew.-%, bevorzugt 0 bis 60 Gew.-% und besonders bevorzugt 0 bis 40 Gew.-% Lösungsmittel;

e) 0 bis 85 Gew.-% Füllstoff.

Dentalmaterialien zur Verwendung als Adhäsiv enthalten vorzugsweise 0 bis 30 Gew.-% Füllstoff und Dentalmaterialien zur Verwendung als Zement oder Füllungsmaterial 20 bis 85 Gew.-% Füllstoff.

Die Erfindung wird im Folgenden anhand von Beispielen näher erläutert.

**Ausführungsbeispiele**

**Beispiel 1: Synthese von Bis-{3-[2-(2-Allyloxy-ethoxymethyl]-2,4,6-trimethylbenzoyl}-phenylphosphinoxid (BAPOmAOEM)**

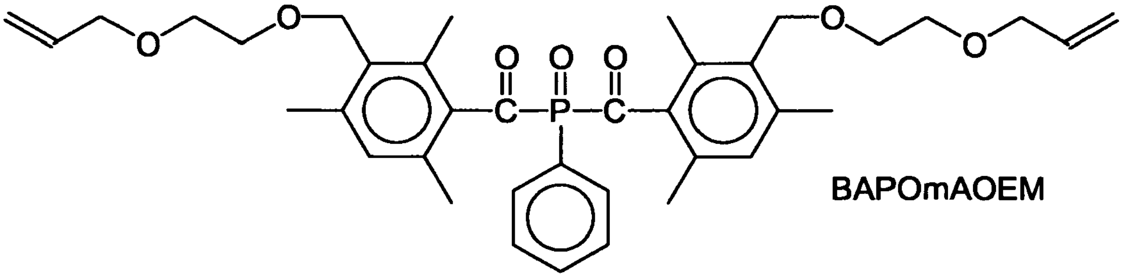

BAPOmAOEM

1. Stufe: 3-(2-Allyloxy-ethoxymethyl)-2,4,6-trimethyl-benzoesäure (AOEMB)

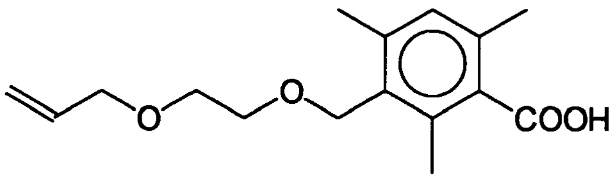

AOEMB

6.70 g (32 mmol) 3-Chlormethyl-2,4,6-trimethylbenzoesäure und 5.65 (101 mmol) KOH wurden in unter Stickstoffatmosphäre vorgelegt. 50 ml (468 mmol) 2-Allyloxyethanol wurden mittels Spritze zugegeben und die Reaktion unter Rühren durch Erhitzen auf 70°C gestartet. Die Reaktionskontrolle erfolgte per Dünnschichtchromatographie, wobei nach 30 min vollständiger Umsatz detektiert wurde. Die Reaktionsmischung wurde anschließend in einen Scheidetrichter überführt und mit 2N Salzsäure auf pH-Wert 1 eingestellt. Das Produkt wurde mit Ethylacetat (EE) (3 x 75 ml) extrahiert und die vereinigten organischen Phasen mit wasserfreiem Natriumsulfat getrocknet. Nach dem Entfernen des Lösungsmittels im Rotationsverdampfer verblieb eine ölige Flüssigkeit. Restliches 2-Allyloxyethanol wurde destillativ (1 mbar, 75°C) abgetrennt und das verbliebene Öl durch mehrmaliges Digerieren mit Hexan gereinigt. Die vereinigten Extrakte ergaben nach Einengen 7.73 g (87 % Ausbeute) an AOEMB als farblosen, wachsartigen Feststoff.
$^1$H-NMR ($CDCl_3$) : $\delta$ (ppm) : 10.28 (s, 1H, -COOH), 6.90 (s, 1H, Ar-$H^5$), 5.98-5.82 (m, 1H, =CH-), 5.22-5.20 (dd, 2H, $H_2C$=), 4.60 (s, 2H, -$CH_2$-O-), 4.04-4.01 (d, 2H, -$CH_2$-O-), 3.65-3.63 (d, 4H, 2x -$CH_2$-O-), 2.43-2.34 (s, 9H, 3x -$CH_3$).

2. Stufe: 3-(2-Allyloxy-ethoxymethyl)-2,4,6-trimethylbenzoylchlorid (AOEMBCl)

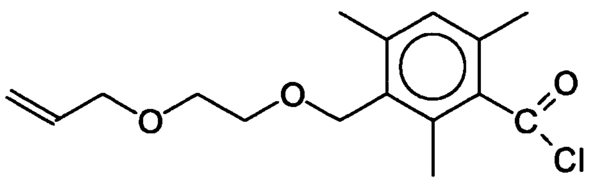

AOEMBCl

7.73 g (0.0278 mol) AOEMB wurden in einen mit Argon gespülten Kolben vorgelegt, der mit einem Septum verschlossen wurde. Danach wurden 77 ml Toluol und 5 Tropfen DMF mittels Spritze zugegeben. Durch langsames Zutropfen von 11.8 ml Oxalsäuredichlorid wurde die Reaktion gestartet (Aufschäumen). Nach 30 min war die Umsetzung vollständig. Über eine Umkehrfritte wurden die ausgefallenen Nebenprodukte unter Argon abgetrennt, schließlich Lösungsmittel und überschüssiges Oxalsäuredichlorid im Rotationsverdampfer abgezogen. Nach Trocknen im Hochvakuum wurden 7.15 g (87 % Ausbeute) an AOEMB als gelber öliger Rückstand erhalten.
$^1$H-NMR: ($CDCl_3$) : $\delta$ (ppm): 6.91 (s, 1H, Ar-$H^5$), 5.90-5.84 (m, 1H, =CH-), 5.32-5.15 (dd, 2H, $CH_2$=), 4.57 (s, 2H, -$CH_2$-O-), 4.03-4.00 (d, 2H, -$CH_2$-O-), 3.66-3.64 (s, 4H, 2x -$CH_2$-O-), 2.43-2.33 (s, 9H, -$CH_3$).

3. Stufe: Bis-{3-[2-(2-Allyloxy-ethoxymethyl]-2,4,6-trimethylbenzoyl}-phenylphosphinoxid (BAPOmAOEM)

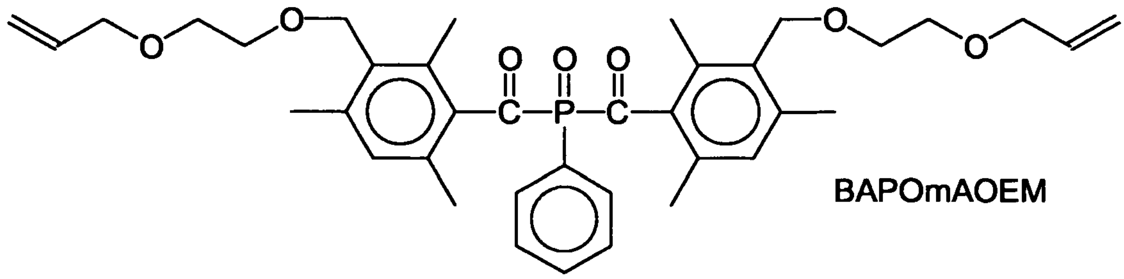

BAPOmAOEM

0.67 g (96.0 mmol) Elementares Lithium wurde direkt in einen Kolben mit Argon und Rührstäbchen eingewogen. Der Kolben wurde mittels eines Septums verschlossen und dazu wurden 32 ml trokkenes THF über eine Spritze zugegeben. Eine Spatelspitze Naphthalin wurde in etwas THF gelöst und ebenfalls zugetropft. Danach wurde für 10 min intensiv gerührt und schließlich 2.88 g (16.0 mmol) P,P-Dichlorphenylphosphin gelöst in 6 ml THF unter intensivem Rühren langsam zugetropft. Aufgrund der großen Exothermie der Reaktion wurde mittels Wasserbad gekühlt. Um das nicht umgesetzte Lithium zu entfernen wurde die schwarze Lösung in einen trockenen argongespülten Kolben übergeführt und hierzu 7.15 g (24.0 mmol) des Säurechlorides AOEMBCl gelöst in 15 ml trockenen THF langsam unter intensivem Rühren zugetropft. Nach Ende der Zugabe wurde noch eine Stunde weitergerührt und anschließend das Lösungsmittel am Rotationsverdampfer entfernt. Der Rückstand wurde in 20 ml Toluol aufgenommen und durch langsames Zutropfen von 1.56 g (16.0 mmol) Wasserstoffperoxidlösung (35 %) unter intensivem Rühren oxidiert, wobei sich das Reaktionsgemisch stark erwärmte (Kühlung im Wasserbad). Nach 20 min wurde das Reaktionsgemisch in 300 ml EE aufgenommen. Die organische Phase wurde mit ges. Natriumhydrogencarbonatlösung (2x50 ml) gewaschen. Die wäßrige Phase wurde mit EE (2x 50 ml) rückextrahiert, die vereinigten organischen Phasen mit gesättigter Kochsalzlösung (Brine) extrahiert und nach dessen Abtrennung mit wasserfreiem Natriumsulfat getrocknet. Die Reinigung des Rohprodukts erfolgte säulenchromatographisch wobei als Laufmittelgemisch Petrolether (PE) : Ethylacetat (EE) = 1 : 1 verwendet wurde. Es ergaben sich 1.9 g (24 % Ausbeute) an BAPOmBAEO als viskoses gelbes Öl.
$^{1}$H-NMR: ($CDCl_3$): δ (ppm): 7.81-7.86 (m, 2H, Ar-H), 7.38-7.51 (m, 3H, Ar-H), 6.83 (s, 2H, Ar-H), 5.78-5.97 (m, 2H, 2x =CH-), 5.12-5.28 (m, 4H, 2x $CH_2$=), 4.48 (s, 4H, 2x Ar-$CH_2$-O-), 3.96-3.99 (d, 4H, 2x =CH-C$\underline{H_2}$-O-), 3.53 (bs, 8H, 4x -$CH_2$-O-), 2.33 (s, 6H, 2x Ar-$CH_3$), 2.17 (s, 6H, 2x Ar-$CH_3$), 2.08 (s, 6H, 2x Ar-$CH_3$).

**Beispiel 2: Herstellung und Untersuchung von Dentalwerkstoffen auf Basis des Bisphenylphosphinoxids aus Beispiel 1**

Zu Untersuchung der Photoinitiatoraktivität und der Lagerstabilität der Photoinitiatoren in Formulierungen wurde eine Zusammensetzung mit den folgenden Komponenten hergestellt. Es handelt sich um eine Zusammensetzungen, die als dentale Primer für Schmelz und Dentin Anwendung findet.

(i) 22 Gew.-% des hydrolysestabilen Vernetzers N,N'-Diethyl-1,3-bis(acrylamido)-propan (DEBAMP);
(ii) 45 Gew.-% des sauren Monomeren 2-[4-(Dihydroxyphosphoryl)-2-oxa-butyl]-acrylsäureethylester (DHPAE) und
(iii) 33 Gew.-% Wasser

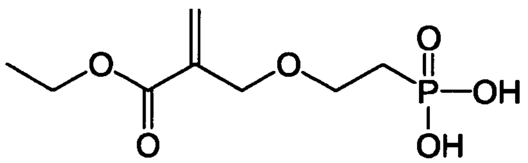

DHPAE

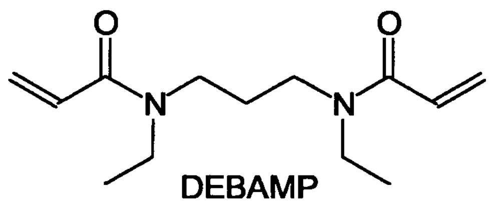

DEBAMP

Zur Herstellung von Modellformulierung wurden von dem in Beispiel 1 beschriebenen Photoinitiator 0.022 mmol in ein Analysenröhrchen eingewogen und mit der obigen Zusammensetzung auf 1.00 g aufgefüllt und unter leichtem

Erwärmen gelöst. Die Aktivität der verwendeten molaren Konzentration des Photoinitiators entsprach in etwa der Aktivität einer 0.8 Gew.-%- Lösung von Camphorchinon/p-Dimethylaminobenzoesäureethylester (CC/DMAB), einem Photoinitiatorsystem das im Dentalbereich verbreitet Anwendung findet.

Für DSC-Messungen wurden schließlich ca. 10 mg der initiatorhaltigen Zusammensetzung in DSC-Schälchen aus Aluminium genau eingewogen und dieses Schälchen auf den rechten Sensor der Meßzelle eines DSC-Gerätes (DSC-50, Shimadzu) gesetzt. Ein leeres Schälchen am linken Sensor diente als Referenz. Die Aufzeichnung des DSC-Gerätes wurde 2.0 min nach dem Aufsetzen des Schälchens gestartet und nach Ablauf von 1.0 min mit der Bestrahlung begonnen. Als Strahlungsquelle diente eine, für den zahnärztlichen Gebrauch bestimmte Dentallampe mit einer Wellenlänge von $\lambda$ = 400-500 nm (Astralis 3, Ivoclar Vivadent). Der Abstand der Lichtquelle zur Probe betrug bei allen Messungen 32 mm. Nach Konstanz der DSC-Linie wurde die Messung abgebrochen. Alle Messungen wurden unter Luft durchgeführt. Eine erste Messung wurde unmittelbar nach der Herstellung der Zusammensetzungen durchgeführt, eine zweite Messung nach 60-tägiger Lagerung der Zusammensetzungen im Dunkeln bei 42 °C. Als Vergleichsinitiatoren wurden auch eine Mischung aus Campherchinon und p-Dimethylaminobenzoesäure und der handelsübliche Initiator [Bis-(2,4,6-Trimethylbenzoyl)-phenylphosphinoxid] (Irgacure 819, Ciba-Speciality Chemicals) untersucht.

Im Ergebnis der DSC-Messung wurde der Zeitpunkt des Wärmeflussmaximums $\mathbf{t_{max}}$ (entspricht der Zeit bis zum Erreichen der höchsten Polymerisationsrate in [s]), die Fläche des Peaks $\Delta\mathbf{H}$ (entspricht der frei gewordenen Polymerisationswärmemenge in [J/g] und die Höhe des Peaks **h** in [mW/mg] bestimmt. Über die Fläche des Peaks $\Delta\mathbf{H}$**,** die Molekulargewichte **MW** der Monomeren und die literaturbekannten theoretischen Polymerisationswärmen der einzelne Komponenten der Zusammensetzungen $\Delta\mathbf{H_0}$**,** die in die nachfolgende Tabelle 1 aufgelistet sind, läßt sich der Doppelbindungsumsatz (DBC) berechnen.

Für einen vollständigen Doppelbindungsumsatz (DBC = 100 %) ergibt sich eine theoretische Polymerisationswärmemenge der Zusammensetzung $\Delta\mathbf{H_{primer}}$ von 207 J/g Zusammensetzung. Der Doppelbindungsumsatz der einzelnen Messung kann damit nach folgender Gleichung (A) berechnet werden:

$$DBC\,[\%] = \frac{\Delta H}{\Delta H_{Primer}} \cdot 100 \qquad \text{(A)}$$

**Tabelle 1**

| Charakteristika der Komponenten der Zusammensetzungen | | | |
|---|---|---|---|
| **Komponente** | **MW[1] [g/mol]** | **$\Delta H_0$[2] [J/mol]** | **Anteil [%] in der Zusammensetzung** |
| DHPAE[3] | 238 | 62.900 | 40 |
| DEBAMP[4] | 238 | 120.600 | 20 |
| Wasser | 18 | 0 | 40 |

[1] Molekulargewicht
[2] theoretische Polymerisationswärme
[3] 2-[4-(Dihydroxyphosphoryl)-2-oxa-butyl]-acryl-säureethylester
[4] N,N'-Diethyl-1,3-bis(acrylamido)-propan

Für beliebige andere Formulierungen kann folgende allgemeine Formel (B) aufgestellt werden:

$$DBC\,[\%] = \frac{\Delta H}{\sum_{x=1}^{n} \frac{\Delta H_0 \cdot w}{MW}} \cdot 100 \qquad \text{(B)}$$

**$\Delta$H** Polymerisationswärme [J/g] (Fläche des Peaks)
**$\Delta H_0$** Theoretische Polymerisationswärme der Einzelkomponente [J/mol]

**w** Gewichtsanteil

Aus der Peakhöhe, der theoretischen Polymerisationswärme und der Dichte des Harzes ρ kann die Polymerisationsrate $R_p$ wie folgt berechnet werden (Formel C):

$$R_P = \frac{h \times \rho}{\Delta H_{0P}} \qquad \text{(C)}$$

**$R_P$** Polymerisationsrate [mol $1^{-1}$ $s^{-1}$]
**h** Höhe des Peaks [mW/mg]
ρ Dichte des Harzes [$\rho_{Primer}$= 1124 g/l]

Die Ergebnisse dieser Berechnungen sind in Tabelle 2 gezeigt. Diese belegen, daß das erfindungsgemäße Bisacylphosphinoxid BAPOmAOEM gegenüber der Verbindung Irgacure 819 nach Lagerung für 60 Tage bei 42 °C eine bessere Reaktivität zeigt. Neben seiner besseren Löslichkeit zeichnet sich der erfindungsgemäße Initiator damit auch durch eine höher Reaktivität und eine höhere Stabilität aus. Ein wesentlicher Vorteil ist, daß der Doppelbindungsumsatz bei der Lagerung konstant bleibt. Ein hoher Doppelbindungsumsatz zeigt eine weitgehende Einbindung des Initiators in das Polymernetzwerk an. Dies ist gleichbedeutend mit einer geringen Menge an nicht eingebundenem und damit auswaschbarem Initiator, was unter dem Gesichtspunkt der Biokompatibilität vorteilhaft ist.

**Tabelle 2**

| **Ergebnisse der Photo-DSC-Messungen** | | | | | | |
|---|---|---|---|---|---|---|
| **Initiator** | **Lagerzeit: 0 Tage** | | | **60 Tage** | | |
| | **$t_{max}$**[1] [s] | **$R_p$**[2] [mol/L s] | **DBC**[3] [%] | **$t_{max}$**[1] [s] | **$R_p$**[2] [mol/L s] | **DBC**[3] [%] |
| **CC/DMAB**[4,*] | 108 | 0.07 | 30 | - | - | - |
| **Irgacure819**[5,*] | 26 | 0.60 | 66 | 29 | 0.52 | 58 |
| **BAPOmAOEM (Bsp. 1)** | 17 | 0.75 | 77 | 23 | 0.65 | 76 |

* Vergleichsbeispiel
[1] Zeitpunkt des Wärmeflußmaximums
[2] Polymerisationsrate gemäß Formel (C)
[3] Doppelbindungsumsatz gemäß Formel (A)
[4] Campherchinon/p-Dimethylaminobenzoesäure
[5] Irgacure 819 wurde aufgrund seiner geringen Löslichkeit in einer Konzentration von 0.005 mmol/g eingesetzt (= maximale Löslichkeit)

## Patentansprüche

1. Polymerisierbarer Dentalwerkstoff enthaltend mindestens ein radikalisch polymerisierbares Monomer, **dadurch gekennzeichnet, daß** er mindestens ein Bisacylphosphinoxid der Formel I enthält,

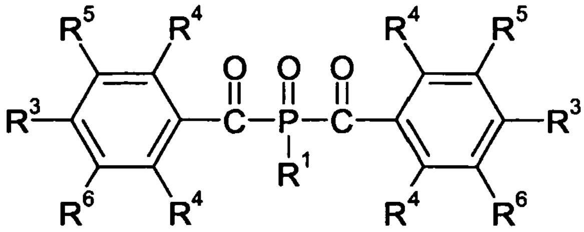

Formel (I)

in der die Variablen unabhängig voneinander die folgenden Bedeutungen haben:

$R^1$ ein linearer oder verzweigter $C_2$ bis $C_{14}$-Alkylrest, der durch ein oder mehrere O-Atome unterbrochen sein kann, PG-Y-$R^2$-X-, ein substituierter oder unsubstituierter, aromatischer $C_6$ bis $C_{14}$-Rest;
$R^2$ entfällt, ein linearer oder verzweigter $C_1$- bis $C_{20}$-Alkylenrest, der durch ein oder mehrere O-Atome unterbrochen sein kann,
$R^3$ H, ein linearer oder verzweigter $C_1$- bis $C_6$-Alkylrest oder PG-Y-$R^2$-X-,
$R^4$ ein linearer oder verzweigter $C_1$-$C_6$-Alkyl- oder -O-$C_1$-$C_6$-Alkylrest,
$R^5$ H oder PG-Y-$R^2$-X-,
$R^6$ H oder PG-Y-$R^2$-X-,
PG eine polymerisationsfähige Gruppe,
X entfällt, O oder S,
Y entfällt, O, S, eine Ester-, Amid- oder Urethangruppe,

wobei das Bisacylphosphinoxid mindestens eine PG-Y-$R^2$-X-Gruppe aufweist und wobei X und/oder Y entfällt wenn $R^2$ entfällt.

2. Dentalwerkstoff nach Anspruch 1, bei dem der aromatische Rest durch eine oder mehrere $C_1$- bis $C_{15}$-Alkoxyreste, die durch ein oder mehrere O-Atome unterbrochen sein können, und/oder PG-Y-$R^2$-X- substituiert ist.

3. Dentalwerkstoff nach einem der vorhergehenden Ansprüche, bei dem die eine oder mehreren polymerisationsfähigen Gruppen unabhängig voneinander aus Vinyl, Allyl, (Meth)acryloyl und/oder Vinylcyclopropyl ausgewählt sind.

4. Dentalwerkstoff nach einem der vorangehenden Ansprüche, bei dem X entfällt und $R^2$ ein linearer oder verzweigter $C_1$- bis $C_{20}$-Alkylenrest ist, der durch ein oder mehrere O-Atome unterbrochen sein kann.

5. Dentalwerkstoff nach einem der vorhergehenden Ansprüche, bei dem mindestens eine der Variablen eine der folgenden Bedeutungen hat:

$R^1$ Phenyl, das unsubstituiert oder durch PG-Y-$R^2$-X- substituiert ist;
$R^2$ -$[CH_2]_n$-$[-O-CH_2-CH_2]_m$- mit n = 1, 2, 3, oder 4 und m = 0, 1,2 oder 3;
$R^3$ H, $C_1$-$C_3$-Alkyl oder PG-Y-$R^2$-X-;
$R^4$ $C_1$-$C_3$-Alkyl oder -O-$C_1$-$C_3$-Alkyl;
$R^5$ H oder PG-Y-$R^2$-X-;
$R^6$ H oder PG-Y-$R^2$-X-;
PG -$CH_2$-CH=$CH_2$ oder -CH=$CH_2$;
X entfällt;
Y entfällt oder O.

6. Dentalwerkstoff nach einem der vorhergehenden Ansprüche, bei dem das Bisacylphosphinoxid 1 bis 5 PG-Y-$R^2$-X-Gruppen enthält.

7. Dentalwerkstoff nach einem der vorhergehenden Ansprüche, der mindestens ein radikalisch polymerisierbares Monomer enthält, das aus monofunktionellen (Meth)acrylaten, wie Methyl-, Ethyl-, Butyl-, Benzyl-, Furfuryl-, Phenyl(meth)acrylat, 2-Hydroxyethyl- und -propyl(meth)-acrylat, Mesitylmethacrylat, 2-(Alkoyxymethyl)acrylsäuren, wie 2-(Ethoxymethyl)acrylsäure, 2-(Hydroxymethyl)acrylsäure, N-mono- oder -disubstituierten Acrylamiden, wie N-Ethylacrylamid, N,N-Dimethacrylamid, N-(2-Hydroxyethyl)acrylamid und N-Methyl-N-(2-hydroxyethyl)acrylamid, N-monosubstituierten Methacrylamiden, wie N-Ethylmethacrylamid und N-(2-Hydroxyethyl)methacrylamid, N-Vinylpyrrolidonen, Allylethern; vernetzenden (Meth)acrylaten, wie Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Butandioldi(meth)acrylat, 1,10-Decandioldi(meth)acrylat und 1,12-Dodecandioldi-(meth)acrylat, Urethane aus 2-(Hydroxymethyl)acrylsäure und Diisocyanten, wie 2,2,4-Trimethylhexamethylendiisocyanat und Isophorondiisocyanat, vernetzende Pyrrolidone, wie z.B. 1,6-Bis(3-vinyl-2-pyrrolidonyl)-hexan, Bisacrylamide, wie Methylen- oder Ethylenbisacrylamid, Bis-(meth)acrylamide, wie N,N'-Diethyl-1,3-bis-(acrylamido)-propan, 1,3-Bis-(methacrylamido)-propan, 1,4-Bis-(acrylamido)-butan, 1,4-Bis-(acryloyl)-piperazin ausgewählt ist.

**8.** Dentalwerkstoff nach einem der vorhergehenden Ansprüche, der mindestens ein radikalisch polymerisierbares Monomer enthält, das aus radikalisch polymerisierbaren Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin, und 4-Vinylbenzoesäure; radikalisch polymerisierbaren Phosphonsäuremonomeren, wie Alkenphosphonsäuren, Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure 2-[4-(Dihydroxyphosphoryl)-2-oxa-butyl]-acrylsäure und 2-[2-Dihydroxyphosphoyl)-ethoxymethyl]-acrylsäure-2,4,6-trimethyl-phenylester; radikalisch polymerisationsfähigen Phosphorsäureestern, wie sind 2-Methacryloyloxypropylmono- oder -dihydrogenphosphat, 2-Methacryloyloxyethylmono- oder -dihydrogenphosphat, 2-Methacryloyloxyethyl-phenyl-hydrogenphosphat, Dipentaerythritolpentamethacryloylöxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat; radikalisch polymerisationsfähigen Sulfonsäuren, wie Vinylsulfonsäure, 4-Vinylphenylsulfonsäure und 3-(Methacrylamido)propylsulfonsäure ausgewählt ist.

**9.** Dentalwerkstoff nach einem der vorhergehenden Ansprüche, der mindestens ein radikalisch polymerisierbares Monomer enthält, das eine oder mehrere Phosphonsäuregruppen oder eine oder mehrere Phosphatgruppen aufweist, wobei diese Gruppen in der Säureform oder in teilweise veresterter Form vorliegen können.

**10.** Dentalwerkstoff nach einem der vorhergehenden Ansprüche, der mindestens einen Füllstoff enthält.

**11.** Dentalwerkstoff nach Anspruch 10, der mindestens einen Füllstoff enthält, der aus amorphen kugelförmigen Materialien auf der Basis von Oxiden, wie $ZrO_2$ und $TiO_2$, nanopartikulären oder mikrofeinen Füllstoffen, wie pyrogene Kieselsäure, nanopartikuläres $Al_2O_3$, $Ta_2O_5$, $Yb_2O_3$, $ZrO_2$, Ag oder $TiO_2$, Mischoxiden von $SiO_2$, $ZrO_2$ und/oder $TiO_2$, Fällungskieselsäure, Minifüllstoffen, wie Quarz-, Glaskeramik- oder Glaspulver mit einer durchschnittlichen Teilchengrösse von 0,01 bis 1 $\mu$m, und röntgenopaken Füllstoffen, wie Ytterbiumtrifluorid oder nanopartikuläres Bariumsulfat, ausgewählt ist.

**12.** Dentalwerkstoff nach einem der vorhergehenden Ansprüche, der mindestens ein Additiv enthält, das aus Stabilisatoren, Aromastoffen, mikrobiociden Wirkstoffen, fluoridionenabgebenden Additiven, optischen Aufhellern, Weichmachern und UV-Absorbern ausgewählt ist.

**13.** Dentalwerkstoff nach einem der vorhergehenden Ansprüche, der

a) 0,001 bis 5 Gew.-%, Bisacylphosphinoxid der Formel (I),
b) 5 bis 80 Gew.-% radikalisch polymerisierbares Monomer,
c) 1 bis 60 Gew.-% acides radikalisch polymerisierbares Monomer,
d) 0 bis 80 Gew.-% Lösungsmittel,
e) 0 bis 85 Gew.-% Füllstoff

enthält.

**14.** Dentalwerkstoff nach Anspruch 13 zur Verwendung als Adhäsiv, der 0 bis 30 Gew.-% Füllstoff enthält.

**15.** Dentalwerkstoff nach Anspruch 13 zur Verwendung als Zement oder Füllungsmaterial, der 20 bis 85 Gew.-% Füllstoff enthält.

**16.** Verwendung eines Dentalwerkstoffs gemäß einem der vorhergehenden Ansprüche zur Herstellung eines Adhäsivs, Beschichtungsmaterial, Zement oder Füllungsmaterials.

**17.** Verwendung eines Dentalwerkstoffs gemäß einem der vorhergehenden Ansprüche als Adhäsiv, Beschichtungsmaterial, Zement oder Füllungsmaterial.

## Claims

**1.** Polymerisable dental material comprising at least one radically polymerisable monomer, **characterised in that** it comprises at least one bisacylphosphine oxide of the Formula I,

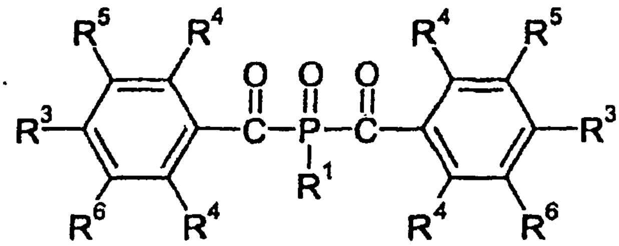

Formula (I)

in which the variables, independently of each other, have the following meanings:

$R^1$ a linear or branched $C_2$ to $C_{14}$ alkyl group that can be interrupted by one or more O atoms, PG-Y-$R^2$-X-, a substituted or unsubstituted aromatic $C_6$ to $C_{14}$ group;
$R^2$ is absent or a linear or branched $C_1$ to $C_{20}$ alkylene group that can be interrupted by one or more O atoms,
$R^3$ H, a linear or branched $C_1$ to $C_6$ alkyl group or PG-Y-$R^2$-X-,
$R^4$ a linear or branched $C_1$ to $C_6$ alkyl or -O-$C_1$-$C_6$ alkyl group,
$R^5$ H or PG-Y-$R^2$-X-,
$R^6$ H or PG-Y-$R^2$-X-,
PG a polymerisable group,
X is absent, O or S,
Y is absent, O, S, an ester, amide or urethane group,

wherein the bisacylphosphine oxide possesses at least one PG-Y-$R^2$-X-group and X and/or Y are absent if $R^2$ is absent.

**2.** Dental material according to claim 1, wherein the aromatic group is substituted by one or a plurality of $C_1$ to $C_{15}$ alkoxy groups that can be interrupted by one or more O atoms, and/or PG-Y-R2-X-.

**3.** Dental material according to one of the previous claims, wherein the one or plurality of polymerisable groups are selected independently of one another from vinyl, allyl, (meth)acryloyl and/or vinylcyclopropyl.

**4.** Dental material according to one of the previous claims, wherein X is absent and $R^2$ is a linear or branched $C_1$ to $C_{20}$ alkylene group that can be interrupted by one or more O atoms.

**5.** Dental material according to one of the previous claims, wherein at least one of the variables has one of the following meanings:

$R^1$ phenyl which is unsubstituted or substituted by PG-Y-$R^2$-X-;

$R^2$ -$[CH_2]_n$-$[-O-CH_2-CH_2]_m$- with n = 1, 2, 3, or 4 and m = 0, 1, 2 or 3;
$R^3$ H, $C_1$-$C_3$ alkyl or PG-Y-$R^2$-X-;
$R^4$ $C_1$-$C_3$ alkyl or -O-$C_1$-$C_3$ alkyl;
$R^5$ H or PG-Y-$R^2$-X-;
$R^6$ H or PG-Y-$R^2$-X-;
PG -$CH_2$-CH=$CH_2$ or -CH=$CH_2$;
X is absent;
Y is absent or O.

**6.** Dental material according to one of the previous claims, wherein the bisacylphosphine oxide comprises 1 to 5 PG-Y-$R^2$-X- groups.

**7.** Dental material according to one of the previous claims comprising at least one radically polymerisable monomer that is selected from monofunctional (meth)acrylates, such as methyl, ethyl, butyl, benzyl, furfuryl, phenyl (meth) acrylate, 2-hydroxyethyl and 2-hydroxypropyl (meth)acrylate, mesityl methacrylate, 2-(alkoxymethyl)acrylic acids, such as 2-(ethoxymethyl)acrylic acid, 2-(hydroxymethyl)acrylic acid, N-mono- or disubstituted acrylamides, such as N-ethyl acrylamide, N,N-dimethacrylamide, N-(2-hydroxyethyl)acrylamide and N-methyl-N-(2-hydroxyethyl)acrylamide, N-mono-substituted methacrylamides, such as N-ethyl methacrylamide and N-(2-hydroxyethyl) methacrylamide, N-vinyl pyrrolidones, allyl ethers; crosslinking (meth)acrylates such as bisphenol-A-di(meth)acrylate, bis-GMA (an addition product of methacrylic acid and bisphenol-A-diglycidylether), UDMA (an addition product of 2-hydroxyethyl methacrylate and 2,2,4-trimethylhexamethylene diisocyanate), di-, tri- or tetraethylene glycol di(meth)acrylate, trimethylolpropane tri(meth)acrylate, pentaerythritol tetra(meth)acrylate, butanediol di(meth)acrylate, 1,10-decanediol di(meth)acrylate and 1,12-dodecanediol di(meth)acrylate, urethanes from 2-(hydroxymethyl)acrylic acid and diisocyanates, such as 2,2,4-trimethylhexamethylene diisocyanate and isophorone diisocyanate, crosslinking pyrrolidones, such as e.g. 1,6-bis(3-vinyl-2-pyrrolidonyl)hexane, bisacrylamides, such as methylene or ethylene bisacrylamide, bis(meth)acrylamides, such as N,N'-diethyl-1,3-bis(acrylamido)propane, 1,3-bis(methacrylamido)propane, 1,4-bis(acrylamido)butane, 1,4-bis(acryloyl)piperazine.

**8.** Dental material according to one of the previous claims, comprising at least one radically polymerisable monomer that is selected from radically polymerisable carboxylic acids such as maleic acid, acrylic acid, methacrylic acid, 2-(hydroxymethyl)acrylic acid, 4-(meth)acryloyloxyethyltrimellitic acid anhydride, 10-methacryloyloxydecylmalonic acid, N-(2-hydroxy-3-methacryloyloxypropyl)-N-phenylglycine, and 4-vinylbenzoic acid; radically polymerisable phosphonic acid monomers, such as alkene phosphonic acids, vinyl phosphonic acid, 4-vinylphenyl phosphonic acid, 4-vinylbenzyl phosphonic acid, 2-methacryloyloxyethyl phosphonic acid, 2-methacrylamidoethyl phosphonic acid, 4-methacrylamido-4-methylpentyl phosphonic acid, 2-[4-(dihydroxyphosphoryl)-2-oxabutyl]acrylic acid and the 2,4,6-trimethylphenyl ester of 2-[2-dihydroxyphosphoryl)ethoxymethyl]acrylic acid; radically polymerisable phosphoric acid esters, such as 2-methacryloyloxypropyl mono- or dihydrogen phosphate, 2-methacryloyloxyethyl mono- or dihydrogen phosphate, 2-methacryloyloxyethylphenyl hydrogen phosphate, dipentaerythritol pentamethacryloyloxyphosphate, 10-methacryloyloxydecyl dihydrogen phosphate, dipentaerythritol pentamethacryloyloxyphosphate, mono-(1-acryloyl-piperidine-4-yl) ester of phosphoric acid , 6-(methacrylamido)hexyl dihydrogen phosphate and 1,3-bis(N-acryloyl-N-propylamino)propane-2-yl dihydrogen phosphate; radically polymerisable sulphonic acids, such as vinyl sulphonic acid, 4-vinylphenyl sulphonic acid and 3-(methacrylamido)propyl sulphonic acid.

**9.** Dental material according to one of the previous claims comprising at least one radically polymerisable monomer that comprises one or a plurality of phosphonic acid groups or one or a plurality of phosphate groups, wherein these groups can be present in the acid form or in the partly esterified form.

**10.** Dental material according to one of the previous claims comprising at least one filler.

**11.** Dental material according to claim 10 comprising at least one filler that is selected from amorphous spherical materials based on oxides, such as $ZrO_2$ and $TiO_2$, nanoparticulate or microfine fillers, such as pyrogenic silica, nanoparticulate $Al_2O_3$, $Ta_2O_5$, $Yb_2O_3$, $ZrO_2$, Ag or $TiO_2$, mixed oxides of $SiO_2$, $ZrO_2$ and/or $TiO_2$, precipitated silica, minifillers, such as quartz, glass ceramic or glass powder with an average particle size of 0.01 to 1 $\mu$m, and X-ray-opaque fillers, such as ytterbium trifluoride or nanoparticulate barium sulphate.

**12.** Dental material according to one of the previous claims comprising at least one additive that is selected from stabilisers, aromatics, microbiocides, fluoride ion-releasing additives, optical brighteners, plasticisers and UV absorbers.

**13.** Dental material according to one of the previous claims comprising

a) 0.001 to 5 wt.% bisacylphosphine oxide of the formula (I),
b) 5 to 80 wt.% radically polymerisable monomer,
c) 1 to 60 wt.% acidic radically polymerisable monomer,
d) 0 to 80 wt.% solvent,
e) 0 to 85 wt.% filler.

**14.** Dental material according to claim 13 for use as an adhesive comprising 0 to 30 wt.% filler.

**15.** Dental material according to claim 13 for use as a cement or filling material comprising 20 to 85 wt.% filler.

**16.** Use of a dental material according to one of the previous claims for the preparation of an adhesive, coating material, cement or filling material.

**17.** Use of a dental material according to one of the previous claims as an adhesive, coating material, cement or filling material.

## Revendications

**1.** Matériau dentaire polymérisable contenant au moins un monomère polymérisable par voie radicalaire, **caractérisé en ce qu'**il contient au moins un bisacylphosphinoxyde de la formule I:

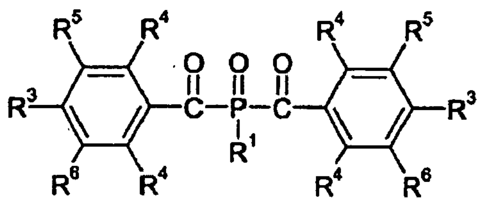

Formule (I)

où les variables ont indépendamment l'une de l'autre, les significations suivantes :

$R^1$ = un reste alkyle en $C_2$ à $C_{14}$, linéaire ou ramifié, qui peut être interrompu par un ou plusieurs atomes O, PG-Y-$R^2$-X-, un reste en $C_6$ à $C_{14}$ aromatique, substitué ou non substitué,
$R^2$ = absent, un reste alkylène en $C_1$ à $C_{20}$, linéaire ou ramifié, qui peut être interrompu par un ou plusieurs atomes O,
$R^3$ = H, un reste alkyle en $C_1$ à $C_6$, linéaire ou ramifié, ou PG-Y-$R^2$-X-,
$R^4$ = un reste alkyle en $C_1$ à $C_6$, linéaire ou ramifié, ou un reste -O-alkyle en $C_1$-$C_6$,
$R^5$ = H ou PG-Y-$R^2$-X-,
$R^6$ = H ou PG-Y-$R^2$-X-,
PG = un groupe polymérisable,
X = absent, O ou S,
Y = absent, O, S, un groupe ester, amide ou uréthanne,

où le bisacylphosphonioxyde comporte au moins un groupe PG-Y-$R^2$-X-, et où X et/ou Y sont absents lorsque $R^2$ est absent.

**2.** Matériau dentaire selon la revendication 1, dans lequel le reste aromatique est substitué par un ou plusieurs restes alcoxy en $C_1$ à $C_{15}$, qui peuvent être interrompus par un ou plusieurs atomes O et/ou PG-Y-$R^2$-X-.

**3.** Matériau dentaire suivant l'une des revendications précédentes, dans lequel le ou les groupes polymérisables sont choisis indépendamment parmi les groupes vinyle, allyle, (méth)acryloyle et/ou vinylcyclopropyle.

**4.** Matériau dentaire suivant l'une des revendications précédentes, dans lequel X est absent et $R^2$ est un reste alkylène en $C_1$ à $C_{20}$, linéaire ou ramifié, qui peut être interrompu par un ou plusieurs atomes O.

**5.** Matériau dentaire suivant l'une des revendications précédentes, dans lequel au moins une des variables a une des significations suivantes :

$R^1$ = phényle, qui est non substitué ou substitué par PG-Y-$R^2$-X-,
$R^2$ = $-[CH_2]_n-[O-CH_2-CH_2]_m-$, avec n = 1, 2, 3 ou 4 et m = 0, 1, 2 ou 3 ;
$R^3$ = H, un reste alkyle en $C_1$ à $C_3$, ou PG-Y-$R^2$-X-,
$R^4$ = un reste alkyle en $C_1$ à $C_3$, ou -O-alkyle en $C_1$-$C_3$,
$R^5$ = H ou PG-Y-$R^2$-X-,
$R^6$ = H ou PG-Y-$R^2$-X-,
PG = $-CH_2-CH=CH_2$ ou $-CH=CH_2$,
X = absent
Y = absent ou O.

**6.** Matériau dentaire suivant l'une des revendications précédentes, dans lequel le bisacylphosphinoxyde contient 1 à 5 groupes PG-Y-$R^2$-X-.

**7.** Matériau dentaire suivant l'une des revendications précédentes, qui contient au moins un monomère polymérisable par voie radicalaire, qui est choisi parmi les (méth)acrylates monofonctionnels, tels que le (méth)acrylate de méthyle, d'éthyle, de butyle, de benzyle, de furfuryle, de phényle, le (méth)acrylate de 2-hydroxyéthyle et -propyle, le méthacrylate de mésityle, des acides 2-(alcoxyméthyl)acryliques, par exemple l'acide 2-(éthoxyméthyl)acrylique, l'acide 2-(hydroxyméthyl)acrylique, des acrylamides N-mono- ou -disubstitués, comme par exemple le N-éthylacrylamide, le N,N-diméthacrylamide, le N-(2-hydroxyéthyl)-acrylamide ou le N-méthyl-N-(2-hydroxyéthyl)acrylamide, et les méthacrylamides N-monosubstitués, comme par exemple le N-éthylméthacrylamide ou le N-(2-hydroxyéthyl)méthacrylamide, ainsi que la N-vinylpyrrolidone et un allyléther ; des (méth)acrylates réticulants, comme le di(méth) acrylate de bisphénol A, le bis-GMA (un produit d'addition d'acide méthacrylique et de bisphénol A-diglycidyléther), l'UDMA (un produit d'addition du méthacrylate de 2-hydroxyéthyle et du 2,2,4-triméthylhexaméthylènediisocyanate), le di(méth)acrylate de di-, tri- ou tétraéthylèneglycol, le tri(méth)acrylate de triméthylolpropane, le tétra(méth)acrylate de pentaérythritol, ainsi que le di(méth)acrylate de butanediol, le di(méth)acrylate de 1,10-décanediol ou le di(méth) acrylate de 1,12-dodécanediol, des uréthannes d'acide 2-(hydroxyméthyl)acrylique et de diisocyanates, comme le 2,2,4-triméthylhexaméthylènediisocyanate ou l'isophoronediisocyanate, des pyrrolidones réticulantes, comme par exemple la 1,6-bis(3-vinyl-2-pyrrolidonyl)hexane, ou des bisacrylamides comme le méthylène- ou l'éthylènebisacrylamide, le bis(méth)acrylamide, comme par exemple le N,N'-diéthyl-1,3-bis(acrylamido)-propane, le 1,3-bis(méthacrylamido)propane, le 1,4-bis(acrylamido)butane ou la 1,4-bis(acryloyl)pipérazine.

**8.** Matériau dentaire suivant l'une des revendications précédentes, qui contient au moins un monomère polymérisable par voie radicalaire, qui est choisi parmi des acides carboxyliques polymérisables par voie radicalaire, comme l'acide maléique, l'acide acrylique, l'acide méthacrylique, l'acide (2-hydroxyméthyl)acrylique, l'anhydride 4-(méth)acryloyloxyéthyltrimellitique, l'acide 10-méthacryloyloxydécylmalonique, la N-(2-hydroxy-3-méthacryloyloxypropyl)-N-phénylglycine et l'acide 4-vinylbenzoïque ; des monomères acide phosphonique polymérisables par voie radicalaire, comme les acides alcènephosphoniques, l'acide vinylphosphonique, l'acide 4-vinylphénylphosphonique, l'acide 4-vinylbenzylphosphonique, l'acide 2-méthacryloyloxyéthylphosphonique, l'acide 2-méthacrylamidoéthylphosphonique, l'acide 4-méthacrylamido-4-méthylpentylphosphonique, l'acide 2-[4-(dihydroxyphosphoryl)-2-oxabutyl]acrylique et l'ester 2,4,6-triméthylphénylique de l'acide 2-[2-(dihydroxyphosphoryl)éthoxyméthyl]-acrylique ; des esters d'acide phosphorique polymérisables par voie radicalaire, comme le mono- et le dihydrogénophosphate de 2-méthacryloyloxypropyle, le mono- et le dihydrogénophosphate de 2-méthacryloyloxyéthyle, l'hydrogénophosphate de 2-méthacryloyloxyéthyle et de phényle, le pentaméthacryloyloxyphosphate de dipentaérythritol, le dihydrogénophosphate de 10-méthacryloyloxydécyle, le pentaméthacryloyloxyphosphate de dipentaérythritol, le monoester (1-acryloylpipéridin-4-yle) de l'acide phosphorique, le dihydrogénophosphate de 6-(méthacrylamido)hexyle et le dihydrogénophosphate de 1,3-bis-(N-acryloyl-N-propylamino)propan-2-yle ; des acides sulfoniques polymérisables par voie radicalaire, comme l'acide vinylsulfonique, l'acide 4-vinylphénylsulfonique ou l'acide 3-(méthacrylamido)propylsulfonique.

**9.** Matériau dentaire suivant l'une des revendications précédentes, qui contient au moins un monomère polymérisable par voie radicalaire, qui présente un ou plusieurs groupes acide phosphonique ou un ou plusieurs groupes phosphate, ces groupes pouvant être présents sous forme acide ou sous forme partiellement estérifiée.

**10.** Matériau dentaire suivant l'une des revendications précédentes, qui contient au moins une charge.

**11.** Matériau dentaire selon la revendication 10, qui contient au moins une charge, qui est choisie parmi des matériaux sphériques amorphes à base d'oxydes, comme $ZrO_2$ et $TiO_2$, des charges nanoparticulaires ou microfines, comme l'acide silicique pyrogéné, $Al_2O_3$, $Ta_2O_5$, $Yb_2O_3$, $ZrO_2$, Ag ou $TiO_2$ nanoparticulaires et respectivement, des oxydes mixtes de $SiO_2$, $ZrO_2$ et/ou $TiO_2$ ou l'acide silicique précipité ainsi que des minicharges, comme la poudre de quartz, de céramique de verre ou de verre, avec une granulométrie moyenne allant de 0,01 à 1 μm, ainsi que des charges opaques aux rayons X, comme le trifluorure d'ytterbium ou le sulfate de baryum nanoparticulaire.

**12.** Matériau dentaire suivant l'une des revendications précédentes, qui contient au moins un additif, qui est choisi parmi des stabilisants, des substances aromatiques, des agents microbicides, des additifs source d'ions fluorure, des azurants optiques, des plastifiants et/ou des agents absorbant les UV.

**13.** Matériau dentaire suivant l'une des revendications précédentes, qui contient

a) 0,001 à 5% en poids de bisacylphosphinoxyde de la formule (I) ;
b) 5 à 80% en poids de monomères polymérisables par voie radicalaire ;
c) 1 à 60% en poids de monomères acides polymérisables par voie radicalaire ;
d) 0 à 80% en poids de solvant,
e) 0 à 85% en poids de charge.

**14.** Matériau dentaire selon la revendication 13, pour une utilisation comme adhésif, qui contient, 0 à 30% en poids de charge.

**15.** Matériau dentaire selon la revendication 13, pour une utilisation comme ciment ou matériau d'obturation, qui contient 20 à 85% en poids de charge.

**16.** Utilisation d'un matériau dentaire selon l'une des revendications précédentes, pour la préparation d'adhésifs, agents de revêtement, ciments ou matériaux d'obturation.

**17.** Utilisation d'un matériau dentaire selon l'une des revendications précédentes, comme adhésif, agent de revêtement, ciment ou matériau d'obturation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 1408265 A
- US 4457818 A
- US 4525256 A
- US 6204302 B
- EP 0334338 A
- EP 0336417 A2
- EP 0173567 A2
- DE 2909992 A1
- EP 1236459 A1
- EP 0948955 A1
- US 20020035169 A1
- DE 3443221 A1
- DE 3801511 A1
- DE 3837569 A1
- US 5399770 A
- DE 19532358 A1
- WO 03019295 A1